Europäisches Patentamt

European Patent Office

Office européen des brevets

(19)

(11) Publication number: **0 120 926**
**B1**

(12) **EUROPEAN PATENT SPECIFICATION**

(45) Date of publication of patent specification: **31.01.90**

(51) Int. Cl.⁵: **A 61 M 16/00**

(21) Application number: **83903329.7**

(22) Date of filing: **22.09.83**

(86) International application number:
**PCT/US83/01472**

(87) International publication number:
**WO 84/01295 12.04.84 Gazette 84/10**

(54) **OXYGEN THERAPY AND APPARATUS.**

(30) Priority: **01.10.82 US 432187**
**21.09.83 US 534378**

(43) Date of publication of application:
**10.10.84 Bulletin 84/41**

(45) Publication of the grant of the patent:
**31.01.90 Bulletin 90/05**

(84) Designated Contracting States:
**BE FR**

(56) References cited:
**US-A-2 208 633**
**US-A-2 535 938**
**US-A-2 535 938**
**US-A-3 045 671**
**US-A-3 973 564**
**US-A-4 054 133**
**US-A-4 120 300**
**US-A-4 192 301**

**Chambers twentieth century dictonary, pages 1398, 1399**

(73) Proprietor: **PHILLIPS, Robert E.**
**12217 Iredell Street**
**Studio City, CA 91601 (US)**
(73) Proprietor: **TIEP, Brian L.**
**632 Norumbega Drive**
**Monrovia, CA 91016 (US)**
(73) Proprietor: **OTSAP, Ben A.**
**7661 Airport Boulevard**
**Los Angeles, CA 90045 (US)**

(72) Inventor: **PHILLIPS, Robert E.**
**12217 Iredell Street**
**Studio City, CA 91601 (US)**
Inventor: **TIEP, Brian L.**
**632 Norumbega Drive**
**Monrovia, CA 91016 (US)**
Inventor: **OTSAP, Ben A.**
**7661 Airport Boulevard**
**Los Angeles, CA 90045 (US)**

(74) Representative: **Lerwill, John et al**
**A.A. Thornton & Co. Northumberland House**
**303-306 High Holborn**
**London, WC1V 7LE (GB)**

Courier Press, Leamington Spa, England.

## Description

The invention described in this specification pertains to a new and improved apparatus which is primarily intended to be utilised in administering oxygen but which are also capable of being used in administering other gases.

One of the oldest methods of delivering oxygen to a patient in order to maintain a given or desired oxygen level in the blood of the patient involves locating an appropriate delivery structure such as a set of nasal cannula immediately adjacent to the nose of the patient and then constantly supplying a metered or controlled supply of oxygen to the delivery structure as the patient inhales and exhales. This method of supplying oxygen is unquestionably highly utilitarian. It is also comparatively undesirable because of the amount or quantity of oxygen which has to be utilised in order to achieve the desired oxygen level of the blood. This is because with this type of procedure significant amounts of oxygen normally escape to the ambient air and are not effectively utilised by the patient.

There are, of course, many variants on the type of procedure indicated in the preceding discussion. Thus, for example, it is well known to substitute for a set of cannula a so-called "tent" which is used to maintain an oxygen enriched atmosphere completely surrounding the head of a patient. It is also known to utilise various mechanical or fluidic switching type mechanisms or structures which are intended only to supply oxygen to a patient during inhalation. Many of such prior structures are constructed so that the patient will re-breathe some or a part of the gas exhaled. One such structure is that disclosed in US—A—2535938 which refers to an apparatus including a mask adapted to cover the mouth and nose of a user, an inflatable bag formed from a flexible tube and connected to the mask, and an oxygen supply nipple for delivering gas into the inflatable bag. During inhalation a quantity of gas is drawn from the bag into the respiratory tract. When the user exhales an initial portion of exhaled gas enters and reinflates the bag, and at the end of the exhalation some gas leaks out between the mask and the user's face. The use of a mask covering the nose and mouth is often undesirable or inconvenient and because all of the gas inhaled is taken from within the structure a bag of comparatively large volume is needed.

Fortunately an understanding of the present invention does not require a detailed discussion of all of the various different known devices of the aforementioned categories. Frequently such devices have been undesirably bulky in character; often they have been of a comparatively complex nature. A result of such complexity, frequently they have been undesirably expensive to manufacture. Often they have been somewhat difficult to use and at other times they have been somewhat unreliable. It is not considered that any of these devices having mechanical or fludiic parts for controlling the delivery of oxygen to a patient have been constructed so as to effectively minimize the amount of oxygen required to maintain a given oxygen level in the blood to a desired extent. As a result of these considerations it is believed that there is a need for improvement in connection with the administration of oxygen. More specifically it is considered that there is a need for a new apparatus for delivering oxygen which minimizes the amount of oxygen required in order to maintain a given or desired oxygen level in the blood. It is also considered that there is a need for an apparatus which is both so simple in character as to be relatively inexpensive to manufacture and to be relatively free from any sort of "mechanical" type problems tending to preclude their utilisation.

Brief summary of the invention

A broad objective of the present invention is to fulfill the needs briefly discussed in the preceding. Thus, the invention is intended to provide a new apparatus for delivering oxygen to a patient while minimizing the amount of oxygen required in order to maintain a desired oxygen level in the blood of the patient. It should be noted that this invention can be utilised to deliver other gases or gas mixtures other than oxygen or oxygen mixtures to a patient although it is not intended for such utilisations.

According to the present invention there is provide an apparatus which is primarily intended for use in oxygen therapy, said apparatus including means for conveying oxygen to the nose of a user of said apparatus, means for supplying oxygen to said means for conveying oxygen, and a structure having an internal volume, at least a portion of which is capable of being changed so as to be increased or decreased in response to a force developed from the movement of gas as a user of said apparatus breathes, said means for conveying being shaped so as to be capable of being located relative to said user's nose so that during breathing cycles exhaled gas will be breathed into said structure in preference to being breathed to the ambient and so that during inhalation gas from within said structure will be inhaled,

said means for supplying being connected to convey oxygen into the interior of said structure in a location in which it will be mixed with gas exhaled into the interior of said structure during exhalation as a result of contact with such gas, characterised in that

said means for conveying comprises a nasal cannular connecting said internal volume with the ambient adjacent to said structure, said cannula being of such dimensions to be incapable of blocking the nostrils of said user,

said changeable portion of the internal volume of said structure has a lesser volume than the volume of gas which is usually moved into or out of the respiratory tract of said user,

and said cannula is shaped so as to be capable of being located within said user's nostrils without closing off said nostrils so that during breathing cycles of exhaled gas will be breathed into said structure in preference to being breathed to the ambient and so that during inhalation gas from within said structure will be inhaled in preference to being inhaled from the ambient, whereby ambient will be inhaled only near the end of an inhalation cycle.

The apparatus of the invention can be relatively simple, normally relatively small and of a shape adapted to a patient's needs, easily and conveniently manufactured at a comparatively nominal cost, and advantageous in that it can be utilised by a patient without the patient manipulating any sort of a movable mechanical structure. Furthermore apparatus embodying the invention can, in accordance with the desire indicated in the preceding discussion, be utilised over a prolonged period with no maintenance.

In using the apparatus, the method carried out during each inhalation-exhalation breathing cycle of the user comprises the following steps:—

at the start of inhalation cycle inhaling a volume of oxygen enriched gas from within the interior of said structure in preference to the inhalation of ambient air while concurrently reducing the internal volume of said structure to an amount corre-.... , sponding to the amount of gas inhaled;

finishing the inhalation by concurrently inhaling ambient air through the space between said person's nostrils and said cannula and gas from within the interior of said structure,

at the start of exhalation using the force developed from the gas exhaled to increase the internal volume of said changeable·portion in an amount corresponding to the volume of oxygen enriched gas previously inhaled during the breathing cycle,

admixing the gas exhaled into said structure with the oxygen introduced into said structure during the entire exhalation cycle so as to form a volume of oxygen enriched gas within said structure for use at the start of inhalation, and

finishing the exhalation cycle by venting to the ambient through the space between said cannula and said person's nostrils any exhaled gas not entering said structure and any gas emitted from said structure as a result of the introduction of oxygen into said structure during this part of the exhalation cycle.

The method involved in using the apparatus of the invention is a new and improved method which is of a comparatively simple character. This method is carried out automatically during breathing once the apparatus has been located in a desired, operative location relative to the nose of a·patient. Due to the ease of practicing or carrying out the method, the apparatus of the invention is especially desirable for use in those circumstances where it is not desirable to restrict the mobility of a patient or in those circumstances when the breathing of the patient will not be monitored, as for example, during sleep.

Brief description of the drawing

Because of the generic nature of this invention it is best more fully described with reference to the accompanying drawing in which:

Figure 1 is a diagrammatic view of one form of an apparatus in accordance with this invention;

Figure 2 is a diagrammatic view of a modified form of an apparatus in accordance with this invention; and

Figure 3 is a diagrammatic view of a further modified form of an apparatus in accordance with this invention.

The various figures shown in the drawing are intended to show the principles or concepts of various different structures or apparatuses embodying the invention. Each of the various items shown is considered to have one or more advantages over each of the other items shown. These advantages frequently—but not always—relate to convenience of use of a specific structure in accordance with a specific person's needs or desires. Because of this it would be inaccurate to refer to any specific one of the apparatuses shown as being always preferable to the others.

The accompanying drawing is not intended to illustrate all of the detailed aspects and features of any specific apparatus of this invention. As will be apparent from the consideration of the appended claims forming a part of this specification this invention pertains to certain generic concepts which may be incorporated within or used within a wide variant of different appearing, differently constructed items of equipment through·the use or exercies of routine engineering skill. Different of such apparatuses have different advantages. As a result some of them more suitable for some applications than others.

Detailed description

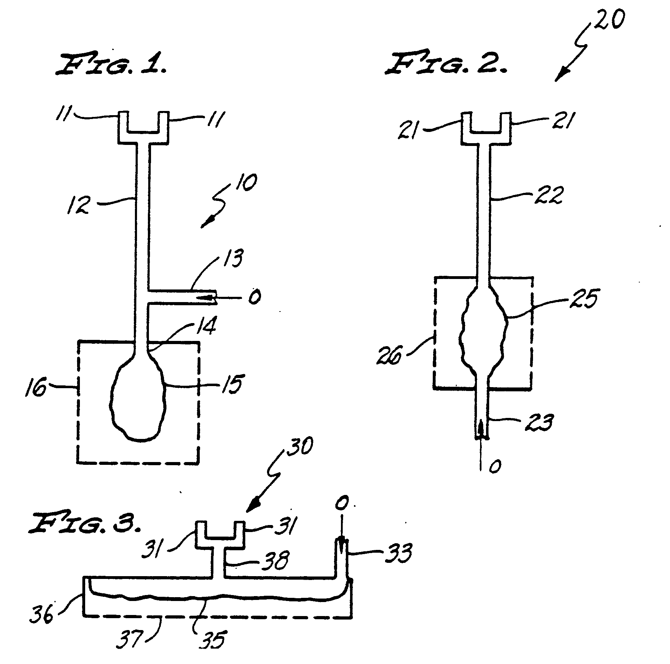

In Figure 1 of the drawing there is shown an apparatus 10 in accordance with this invention which includes a set or pair of conventional nasal cannula 11 both of which are connected to an elongated conduit 12. This conduit 12 is provided with an inlet or inlet line 13 which is adapted to be utilized in continuously supplying oxygen to the apparatus 10 at a constant rate. Normally this line 13 will be connected to a tank of compressed oxygen (not shown) or other oxygen source through a usual flow control valve (not shown) and pressure regulator (not shown).

The extremity 14 of the conduit 12 remote from the cannula 11 is connected to a very loose, floppy type, exceedingly light weight impervious diaphragm 15 which may, as in the apparatus 10, take the shape of a floppy bag. This diaphragm preferably is formed of a thin, impervious, light weight material or synthetic rubber. It may or may not be slightly elastic in character. This bag is normally protected by a perforated housing 16 serving to prevent it from being damaged.

The nature of this diaphragm 15 is quite important with the present invention. It can be compared to a theoretical frictionless, weightless piston in a cylinder because of its function in the apparatus 10. It must be capable of being expanded and contracted so as to change in volume by the forces developed from the movement of gas caused by a user of the apparatus 10 breathing. The force involved in such movement is normally less than that developed or manifested at the nostrils of the user (not shown) by an amount corresponding to the loss of any energy by the movement of gas through and/or within the apparatus 10. It is considered that this is best explained by discussing the "use" of the apparatus 10.

As this apparatus 10 is used the cannula 11 are inserted within the nostrils of the user (not shown) so as to be in a position such that exhaled gas will flow into the cannula 11 and so that during inhalation gas will be inhaled from the apparatus 10 through these cannula 11. The cannula 11 must not completely block or close the nostrils of the user. Instead they must be spaced relative to the nostrils of the user so as to permit flow around them or between them and the interiors of the nostrils of a user (not shown) as the apparatus 10 is used. Normally the achievement of such flow presents no problem when the usual round cannula are used because of the fact that the nostrils of the person are of a non-round configuration.

The manner in which the cannula 11 fit is quite important in achieving that is referred to herein as preferential flow. When the apparatus 10 is initially to be used, not only are the cannula 11 positioned as indicated, but in addition oxygen at a very low flow rate as subsequently discussed will be supplied through the line 13. This will tend to fill the apparatus 10 and to expand the diaphragm 15 so as to increase the internal volume within the apparatus 10. As the person using the apparatus 10 initially inhales at this point the person will inhale a quantity of gas which approximately corresponds to the volume to which the diaphragm 15 has been expanded. Of course, this volume will also correspond to the volume to which the diaphragm can be collapsed.

Because of the position of the cannula 11 at the start of inhalation the person will inhale this volume of gas from within the apparatus 10 preferentially to inhaling any ambient air from around the cannula 11. It is important that this diaphragm 15 move or collapse quite easily so as to permit what may be regarded as a bolus or oxygen enriched gas to be the first gas inhaled by the user at the start of inhalation. This initially inhaled gas will tend to be that gas which reaches the lowermost part of the respiratory track in the lungs where oxygen is transferred to the blood.

When this initial bolus or quantity of gas from within the apparatus 10 is within the respiratory track the only additional gas which can be drawn into the respiratory track from the apparatus 10 by the breath during the remainder of the inhalation part of the breathing cycle will correspond to the volume of oxygen delivered to the apparatus through the line 13. As this limited or restricted amount of gas is drawn into the body through the cannula 11 the additional and principal gas necessary to fill the respiratory track will be drawn in from around the cannula 11 until such time as the breathing cycle changes to exhalation. The flow of oxygen must not be at a pressure or at a volume which would be capable of causing inflation of the diaphragm 15 during this last part of the inhalation cycle.

On exhalation the initial gas exhaled will be that generally within the upper portion of the upper respiratory track which has not reached the lungs. It will contain substantially the oxygen content of the gas inhaled during the last part of inhalation. This oxygen content will, of course, be that of the ambient air as enriched by gas displaced from the conduit 12 by the oxygen travelling into the apparatus 10 through the line 13 during the last part of inhalation. This initial quantity or bolus of exhaled gas will, of course, be captured within the apparatus 10 as the diaphragm 15 expands.

It is noted that the gas which accumulates within the diaphragm 15 during this initial part of exhalation will normally include and indeed may consist entirely of gas which remained within the apparatus 10 at the close of the inhalation cycle. The fact that this gas within the diaphragm 15 may not be that captured on exhalation is immaterial so long as the apparatus 10 is constructed so that those internal portions of it (not separately numbered) closest adjacent to the cannula 11 contain largely air from the ambient which has not reached the lungs enriched slightly with oxygen.

During exhalation as the apparatus 10 is being filled as noted with exhaled air oxygen will, of course, be flowing into this apparatus 10. In order to achieve such flow it is necessary that the oxygen being moved into the apparatus 10 be at a pressure greater than the pressure of the exhaled gas during normal breathing. The oxygen that flows into this apparatus 10 during this initial part of the exhalation cycle will tend to mix with the gas already within the apparatus 10 and that exhaled into it. This mixing will, of course, be accompanied by a gradual oxygen enrichment of the gas within the apparatus 10 during the initial part of the exhalation cycle.

After the apparatus 10 tends to become full and after the diaphragm 15 has expanded during exhalation, additional flow into the apparatus 10 from the respiratory tract of the user is prevented and as a result the additional exhaled gas will be vented to the ambient around the cannula 11. It is noted that this last gas exhaled to the ambient will tend to be diluted somewhat by a gas mixture from within the conduit 12 as a result of displacement caused by the continuous flow of oxygen through the line 13. Further this last gas exhaled will contain the principal byproduct gas of the breathing cycle—carbon dioxide. As a consequence of this these gases will not remain in the "system" consisting of the apparatus 10 and the respiratory track of the user of this apparatus 10.

At this point the user of the apparatus 10 will normally commence the next respiratory cycle by inhalation. This will result in the user inhaling an oxygen enriched bolus or quantity of exhaled gas from within the conduit 12 adjacent to the cannula 11 until such time as the diaphragm 15 has been collapsed. At this point the cycle of operations indicated in the preceding will continue indefinitely as long as the apparatus 10 is used.

In order for an apparatus 10 to function in the manner indicated in the preceding discussion it is necessary for the volume change caused by the movement of the diaphragm 15 to be less than the volume of gas which is normally moved into or out of the respiratory tract of a user of this apparatus. It is considered that it would be inefficient to use a volume of this latter size and that it is preferable to use an apparatus 10 constructed so that this volume is no greater than about the volume required in the respiratory tract to cover the alveolar membranes during inhalation. This will, of course, vary from patient to patient. The objective of the use of such a volume is to make reasonably certain that an effective amount of oxygen for oxygen therapy reaches only those parts of the lungs where oxygen transference takes place. This is difficult to achieve because of the fact that some gas will remain in the respiratory tract after expiration.

Of course the higher the oxygen content of the bolus of gas taken into the respiratory tract at the start of inhalation and the larger the size of this bolus the greater the amount of oxygen which can be expected to reach those portions of the lungs where the alveolar membranes are located. The volume of gas which is supplied to a user at the start of inhalation with the apparatus 10 should be limited in order to insure preferential movement of gas from within the apparatus at the start of inhalation. If this volume is greater than about 60 ml. normally the inertia of the gas and the diaphragm 15 will be so great that the entire content of this volume will not be "snapped" into the respiratory tract on the start of inhalation, but instead will be sufficiently large to delay flow from the apparatus 10 to a user to such an extent that the bolus of oxygen enriched gas will be significantly diluted by ambient air flowing around the exteriors of this cannula 11.

When this occurs the initially inhaled gas tending to reach the alveolar membranes will not have as high an oxygen content as reasonably possible. On the other hand if the volume capable of being taken up by or delivered by the apparatus 10 is so small it is considered that it will contain an amount of oxygen which is too small to be effective in oxygen therapy—regardless of the oxygen content of this volume. As a consequence of this it is considered that the apparatus 10 should be constructed so as to be capable of delivering at least 8 ml. of any oxygen enriched gas mixture. It is presently considered that preferred results are achieved when this value of oxygen enriched gas is within the range from about 10 to about 20 ml. since such a quantity of gas can be easily and rapidly moved through the use of the apparatus 10 without being accompanied by a significant movement of collateral ambient air.

For such a volume to be effective in oxygen therapy it is necessary that its oxygen content be as high as can be reasonably achieved in the apparatus 10. The total oxygen content of the volume within the apparatus 10 will, of course, vary in accordance with this volume and the rate at which oxygen is introduced into it as well as in accordance with the manner in which the exhaled gas captured in the apparatus 10 is mixed with and displaced from the apparatus as the oxygen flows into the apparatus 10. To obtain a bolus of oxygen enriched gas in the apparatus 10 without any noticeable waste of the oxygen it is believed necessary for the internal volume within the apparatus between the oxygen inlet and the ends (not numbered) of the cannula 11 to be at least as great as the volume to be delivered to a user on inhalation. This will normally correspond to the volume change within the apparatus caused by either the expansion or the contraction of the diaphragm 15.

In general it is considered that most effective utilization of oxygen occurs when the oxygen flow rate is such that during the latter part of the exhalation cycle the quantity of gas to be delivered on inhalation located adjacent to and within the cannula will be enriched with oxygen so as to have an oxygen content of at least 50% but less than 100% by weight. If the latter degree of enrichment is sought there is danger of oxygen being unnecessarily lost to the ambient air. It is presently considered that preferred results are achieved when the oxygen content of the bolus of gas to be delivered at the start of inhalation is from about 80 to about 98% by weight.

In Figure 2 of the drawing there is shown a modified apparatus 20 of the present invention in which a diaphragm 25 corresponding to the diaphragm 15 in the form of a sleeve is located generally between an oxygen inlet 23 and a conduit 22 carrying cannula 21. In this apparatus 20 a perforated canister-like housing 26 serves to protect the diaphragm 25 and may also serve to prevent it expanding to an undesired extent.

In Figure 3 of the drawing there is shown a further modified apparatus 30 of the present invention which includes an elongated cup-like housing 36 having a perforated wall 37. A diaphragm 35 corresponds to the diaphragm 15 is secured in this housing 36 so as to enclose that portion of the housing (not separately numbered) of an imperforate character. An inlet line 33 leads into the housing 36 and an outlet 38 is provided so as to lead it to cannula 31. If desired these cannula may be located so as to extend directly from the housing 36. This apparatus 30 is of such a nature that in use the diaphragm will, at the end of inhalation, close off, or substantially close off the outlet 38 from the inlet 33 until it is moved by incoming oxygen. With this structure the expansion of the diaphragm 35 can be controlled by either its construction or by the geometry of the housing 36.

All of these apparatuses 10, 20 and 30 operate or are operated in substantially the same manner. As a consequence of this it is not considered necessary to discuss their operation in detail.

It is possible to modify the operation of the apparatus 10 or any of the other different apparatuses discussed to a slight or limited extent by being sure that the diaphragm 15 or the corresponding diaphragm used in any of these other apparatuses is slightly elastic and is capable of stretching slightly in response to the exhaled breath of a user. With this type of construction the rapid change in pressure within the apparatus 10 or any of the other apparatuses described on inhalation will enable any such diaphragm to return to its initial configuration as a result of the energy stored within it. This is considered to be slightly beneficial as facilitating inhalation.

## Claims

1. An apparatus which is primarily intended for use in oxygen therapy, said apparatus including means (11; 21; 31) for conveying oxygen to the nose of a user of said apparatus, means (13; 23; 33) for supplying oxygen to said means for conveying oxygen, and a structure (12, 15; 22, 25; 35, 38) having an internal volume, at least a portion of which is capable of being changed so as to be increased or decreased in response to a force developed from the movement of gas a user of said apparatus breathes, said means for conveying being shaped so as to be capable of being located relative to said user's nose so that during breathing cycles exhaled gas will be breathed into said structure in preference to being breathed to the ambient and so that during inhalation gas from within said structure will be inhaled,

said means for supplying being connected to convey oxygen into the interior of said structure in a location in which it will be mixed with gas exhaled into the interior of said structure during exhalation as a result of contact with such gas, characterised in that

said means for conveying comprises a nasal cannula (11; 21; 31) connecting said internal volume with the ambient adjacent to said structure, said cannula being of such dimensions to be incapable of blocking the nostrils of said user,

said changeable portion of the internal volume of said structure has a lesser volume than the volume of gas which is usually moved into or out of the respiratory tract of said user,

and said cannula (11; 21; 31) is shaped so as to be capable of being located within said user's nostrils without closing off said nostrils so that during breathing cycles the exhaled gas will be breathed into said structure in preference to being breathed to the ambient and so that during inhalation gas from within said structure will be inhaled in preference to being inhaled from the ambient, whereby ambient will be inhaled only near the end of an inhalation cycle.

2. An apparatus as claimed in claim 1 wherein: said changeable portion of said volume can be changed in amounts of from about 8 to about 60 ml. and

the internal volume between said means for supplying and said means for conveying is from about 8 to about 60 ml.

3. An apparatus as claimed in claim 4 wherein: said changeable portion of said volume can be changed in an amount of from about 10 to about 20 ml. and

the internal volume between said means for supplying said means for conveying is from about 8 to about 60 ml.

## Patentansprüche

1. Eine Vorrichtung, die primär zur Verwendung bei der Sauerstofftherapie vorgesehen ist, mit einer Einrichtung (11; 21; 31) für die Leitung von Sauerstoff in die Nase eines Benutzers der Vorrichtung, einer Einrichtung (13; 23; 33) für die Zuführung von Sauerstoff zu der Einrichtung für die Leitung von Sauerstoff, und einer Anordnung (12, 15; 22, 25; 35, 38) mit einem internen Volumen, von dem wenigstens ein Teil dazu in der Lage ist, geändert zu werden, so daß es in Reaktion auf eine Kraft, die sich durch die Bewegung eines Gases, wie es ein Benutzer der Vorrichtung atmet, entfaltet, vergrößert oder verkleinert wird, wobei die Einrichtung für die Leitung so ausgestaltet ist, daß sie relativ zur Nase des Benutzers derart angeordnet werden kann, daß während Atmenzyklen ausgeatmetes Gas bevorzugt in die Anordnung und nicht in die Umgebung geatmet und daß während des Einatmens Gas aus dem Inneren der Anordnung eingeatmet wird, wobei die Einrichtung für die Zuführung zur Leitung von Sauerstoff in das Innere der Anordnung an einer Stelle angeschlossen wird, an der es mit während der Ausatmung in das Innere der Anordnung ausgeatmetem Gas im Ergebnis eines Kontakts mit diesem Gas vermischt wird, dadurch gekennzeichnet, daß die Einrichtung zur Leitung eine Nasenkanüle (11; 21; 31), umfaßt, die das interne Volumen mit der an die Anordnung angrenzenden Atmosphäre verbindet und solche Abmessungen aufweist, daß sie die Nasenlöcher eines Benutzers nicht blockieren kann, daß der veränderbare Teil des internen Volumens der Anordnung ein Volumen aufweist, das geringer als das Gasvolumen ist, das gewöhnlich in den oder aus dem Atemtrakt eines Benutzers bewegt wird, und daß die Kanüle (11; 21; 31) so geformt ist, daß sie in den Nasenlöcher des Benutzers ohne die Nasenlöcher abzuschließen angeordnet werden kann, so daß während Atemzyklen das ausgeatmete Gas vorzugsweise in die Anordnung und nicht in die Umgebung geatmet und so daß während des Einatmens bevorzugt Gas aus dem Innern der Anordnung und nicht aus der Umgebung eingeatmet wird, wobei aus der Umgebungatmosphäre nur kurz vor dem Ende eines Einatmungszyklus eingeatmet wird.

2. Eine Vorrichtung nach Anspruch 1, wobei der veränderbare Teil des Volumens um Beträge zwischen 8 und 60 ml geändert werden kann und das interne Volumen zwischen der Einrichtung zur Zuführung und der Einrichtung zur Leitung zwischen 8 und 60 ml beträgt.

3. Eine Vorrichtung nach Anspruch 4, wobei der veränderbare Teil des Volumens um einen Betrag zwischen 10 und 20 ml geändert werden kann und das interne Volumen zwischen der Einrichtung zur Zuführung und der Einrichtung zur Leitung zwischen 8 und 60 ml beträgt.

**Revendications**

1. Appareil essentiellement destiné à être utilisé en oxygénothérapie, l'appareil comprenant un dispositif (11; 21; 31) destiné au transport d'oxygène vers le nez d'un utilisateur de l'appareil, un dispositif (13; 23; 33) de transmission d'oxygène au dispositif de transport d'oxygène, et une structure (12, 15; 22, 25; 35, 38) ayant un volume interne, une partie au moins de ce volume pouvant changer afin qu'elle augmente ou diminue sous l'action d'une force due au déplacement du gaz lorsqu'un utilisateur de l'appareil aspire, le dispositif de transport ayant une configuration permettant sa disposition, par rapport au nez de l'utilisateur, d'une manière telle que, pendant les cycles de respiration, le gaz expiré est chassé préférentiellement dans la structure au lieu d'être chassé à l'atmosphère ambiante, et que, pendant l'aspiration, le gaz de l'intérieur de la structure est aspiré,

le dispositif de transmission étant connecté afin qu'il transporte de l'oxygène à l'intérieur de la structure à un emplacement auquel il peut se mélanger au gaz expiré à l'intérieur de la structure pendant l'expiration, à la suite du contact avec ce gaz, caractérisé en ce que

le dispositif de transport comporte une canule nasale (11; 21; 31) reliant le volume interne à l'atmosphère ambiante près de la structure, la canule ayant des dimensions telles qu'elle ne peut pas boucher les narines de l'utilisateur,

la partie de volume interne de la structure qui peut changer a un volume inférieur au volume de gaz qui est habituellement déplacé vers le système respiratoire de l'utilisateur ou hors de ce système respiratoire, et

la canule (11; 21; 31) a une configuration permettant sa disposition dans les narines de l'utilisateur sans fermeture de ses narines si bien que, pendant les cycles respiratoires, le gaz expiré est chassé préférentiellement dans ladite structure au lieu d'être chassé à l'atmosphère ambiante, et que, pendant l'aspiration, le gaz de l'intérieur de la structure est aspiré préférentiellement à l'aspiration du gaz ambiant, si bien que le gaz ambiant n'est aspiré qu'à la fin d'un cycle d'inhalation.

2. Appareil selon la revendication 1, dans lequel

la partie du volume qui peut changer peut changer d'une valeur comprise entre environ 8 et 60 cm$^3$, et

le volume interne, compris entre le dispositif de transmission et le dispositif de transport, est compris entre environ 8 et 60 cm$^3$.

3. Appareil selon la revendication 2, dans lequel

la partie du volume qui peut changer peut changer d'une valeur comprise entre environ 10 et 20 cm$^3$, et

le volume interne, compris entre le dispositif de transmission et le dispositif de transport, est compris entre environ 8 et 60 cm$^3$.

*FIG. 1.*

*FIG. 2.*

*FIG. 3.*